Europäisches Patentamt

European Patent Office

Office européen des brevets

(11) Numéro de publication: **0 463 937 B1**

(12) **FASCICULE DE BREVET EUROPEEN**

(45) Date de publication de fascicule du brevet: **12.10.94**  (51) Int. Cl.⁵: **A61K 7/06**, A61K 7/48

(21) Numéro de dépôt: **91401653.0**

(22) Date de dépôt: **19.06.91**

(54) **Lotion pour le traitement de l'alopécie.**

(30) Priorité: **26.06.90 FR 9008002**

(43) Date de publication de la demande:
**02.01.92 Bulletin 92/01**

(45) Mention de la délivrance du brevet:
**12.10.94 Bulletin 94/41**

(84) Etats contractants désignés:
**AT BE CH DE DK ES FR GB GR IT LI LU NL SE**

(56) Documents cités:
**FR-A- 2 653 996
GB-A- 801 726
GB-A- 1 012 585
GB-A- 2 170 405
US-A- 3 519 711**

(73) Titulaire: **JOUVEINAL S.A.
Tour Maine Montparnasse
33 Avenue du Maine
F-75755 Paris Cedex 15 (FR)**

(72) Inventeur: **Chamoux, Catherine
12 Avenue de la Tranquilité
F-78000 Versailles (FR)**
Inventeur: **Doat, Bernard
67 Bd Jacques Millot
F-49100 Angers (FR)**

(74) Mandataire: **Bourgognon, Jean-Marie et al
Cabinet Flechner
22, Avenue de Friedland
F-75008 Paris (FR)**

Rank Xerox (UK) Business Services
(3.10/3.09/3.3.3)

**Description**

La présente invention se rapporte à l'application d'une lotion pour prévenir la chute des cheveux ou pour les faire repousser à l'exception d'une application à titre de méthode de traitement du corps humain ou animal et de méthode de diagnostic appliquée au corps humain, et à des lotions nouvelles convenant d'ailleurs, d'une manière générale, aux soins de l'épiderme.

Au GB-A-2 170 405, on décrit l'application de la résorcérine pour favoriser la croissance capillaire. Au GB-A-801 726, on décrit une composition aqueuse contenant de l'acide borique et de l'acide salicylique pour une application topique à la peau.

La diminuation progressive ou subite de la densité capillaire du cuir chevelu correspond à des alopécies plus ou moins localisées et étendues qui, à terme, établissent une calvitie. La nature des alopécies, leurs origines et leurs causes sont des plus diverses (HINCKY M., Les alopécies - Encycl. Méd. Chir., Paris. Dermatologie, 12855 A 10, 2-1981), et il est proposé d'appliquer des substances diverses mises sous formes liquides afin d'éviter la chute des cheveux et favoriser leur repousse et leur croissance. Les lotions destinées à ces frictions locales contiennent par exemple des goudrons comme le groudron de houille ou l'huile de cade, des vasodilatateurs comme le chlorhydrate d'histamine ou encore, et plus récemment, un antihypertenseur tel que le Minoxidil (DCI).

Ces applications sont de longue durée et d'efficacité incertaine.

Certaines sont onéreuses et leur utilisation est souvent assujettie à des recommandations et précautions d'emploi concernant par exemples le strict respect d'une périodicité du traitement, les conditions de conservation particulières des solutions ou encore le risque de souillure indélébile du linge ou des vêtements par les solutions.

Plus gravement, leur utilisation incontrôlée et abusive peut provoquer des effets secondaires indésirables tels qu'irritations, inflammations, rougeurs et enfin couperoses locales permanentes.

Remédiant avantageusement à ces inconvénients, on a trouvé un procédé qui consiste à appliquer sur la zonealopécique à traiter une lotion aqueuse d'acide borique, d'acide salicylique et à titre de composé phénolique uniquement un ou plusieurs monophénols.

A cet effet, les solutions aqueuses utilisées dans l'invention contiennent généralement en poids, par rapport au poids total de la solution, de 0,3 à 3,0 % d'acide borique et de 0,1 à 1,0 % d'acide salicylique, la proportion relative entre ces constituants étant de deux à vingt parties d'acide borique pour une partie d'acide salicylique, et de 0,001 à 0,1 % d'un ou plusieurs monophénols. On les prépare par mélange de leurs constituants à de l'eau.

Individuellement, ou sous forme de mélanges de deux de ces constituants, les solutions aqueuses obtenues sont inactives sur l'évolution des alopécies, contrairement à celles contenant l'association des trois produits.

Ainsi, des frictions biquotidiennes durant un mois avec une solution à 1 % en poids d'acide borique s'avère sans effet sur la chute des cheveux d'un sujet atteint d'alopécie séborrhéïque diffuse.

Des frictions identiques avec une solution à 0,3 % d'acide salicylique conduit aussi à un échec, et il en est de même avec ces deux solutions auxquelles on ajoute 0,01% de p-crésol ou encore avec une solution contenant 1 % d'acide borique et 0,3 % d'acide salicylique.

Par contre, dans un cas d'alopécie semblable, une lotion aqueuse comprenant,en poids par rapport au poids total,1 % d'acide borique, 0,3 % d'acide salicylique et 0,01 % de p-crésol permet de stopper pratiquement la chute des cheveux, l'observation étant objectivée par la détermination de la densité capillaire d'une surface déterminée dans la zone du vortex du sujet avant et après les frictions.

Dans ces solutions, le p-crésol est représentatif des composés monophénoliques essentiels à la composition.

A la différence des polyphénols dont l'utilisation s'avère délicate sinon néfaste du fait de leur activité décolorante, notamment pour les chevelures de couleur claire, ces monophénols sont désinfectants et éliminent l'éventuelle flore bactérienne transitoire pathogène notamment celle des orifices folliculaires, effet qui est obtenu avec de 0,001 à 0,1 % en poids de phénol, de p-crésol ou de ses isomères ou encore d'une composition dont ils représentent les constituants majeurs comme dans le lysol.

L'efficacité de cette lotion est avantageusement renforcée par l'addition d'un agent astringent que l'on choisit parmi les sels d'aluminium tels que l'hydroxychlorure, le sulfate, le phénolsulfate ou bien le phénolsulfate de zinc ou encore des extraits végétaux riches en tanins comme ceux de l'écorce d'Hamamélis et, plus particulièrement, l'acide tannique qui est préféré à une concentration de 0,01 à 0,10 % en poids dans la solution ce qui lui confère des propriétés cytoprotectrices et anti-exudatives.

Egalement, l'addition d'excipients contribue à rendre l'utilisation répétée des lotions plus agréable.

Dans ce but, on y ajoute un agent émollient, comme la glycérine, à une concentration de 0,01 à 0,1 % en poids, et des composés ou des compositions parfumées. Entre autres exemples de compositions discrètement parfumées non persistantes, on préfère un mélange de menthol et d'eucalyptol à raison de quelques milligrammes par litre auquel on ajoute une trace de teinture d'eucalyptus.

Selon l'invention, les lotions les plus adaptées pour favoriser la repousse des cheveux et/ou éviter ou stopper leur chute sont celles qui contiennent, en poids par rapport au poids total de la lotion, de 0,50 à 1,50 % d'acide borique et 0,15 à 0,75 % d'acide salicylique au titre de constituants essentiels et, au titre d'adjuvants, de 0,015 à 0,075 % d'acide tannique et de 0,005 à 0,1 % d'un désinfectant composé d'un monophénol approprié et éventuellement de lysol.

L'invention, consiste à appliquer de une à quatre fois par jour la lotion sur la zone alopécique à traiter à raison de 1 à 2 ml par dm$^2$.

Dans le cas d'alopécie partielle afin de stopper la chute des cheveux la solution est appliquée par friction, dans les cas d'alopécies totales afin de provoquer la repousse des cheveux on applique localement un tampon de coton ou une compresse imprégnée par la solution.

La durée d'application est tributaire du degré d'avancement de l'état alopécique et de l'importance de la surface à traiter. Ainsi, dans le cas d'alopécie diffuse, l'efficacité peut être constatée après trois à quatre semaines. Elle est objectivée par une diminution évidente de la perte des cheveux ou d'une manière plus rigoureuse par les résultats de trichogrammes pratiqués périodiquement sur des mèches de cheveux représentatives de la zone à traiter. Dans ces analyses, il est alors constaté, à l'examen microscopique des racines une augmentation significative du pourcentage des follicules en phase de croissance dite "phase anagène" alors qu'inversement le pourcentage des follicules en phase terminale d'activité dite "phase télogène" diminue. Dans les cas d'alopécies totales localisées, la réactivation des follicules capillaires se traduit par l'apparition d'un duvet dont la consistance et l'aspect évolue en poursuivant le traitement, et aboutit à terme à la formation des cheveux de couleur identique à celle de la coloration naturelle du sujet.

Certaines des lotions utilisées dans le procédé suivant l'invention sont nouvelles. Ce sont celles dans lesquelles le monophénol désinfectant est uniquement le p-crésol à une concentration,en poids par rapport au poids total de la lotion, de 0,005 à 0,1%.

Une lotion nouvelle particulièrement préférée pour l'effet visé est celle qui comprend en poids par rapport à son poids total de 0,50 à 1,20 % d'acide borique, de 0,10 à 0,50 % d'acide salicylique, 0,010 à 0,050 % d'acide tannique et de 0,010 à 0,050 % de para crésol associés à une solution glycéro-éthanolique de menthol et d'eucalyptol à raison de 0,01 à 0,10 %.

Ces lotions sont aussi appropriées aux soins de l'épiderme comme au traitement des peaux dites grasses, des petits boutons et des points noirs, de même que pour traiter les affections inflammatoires bénignes comme les irritations, les feux du rasoir, les rougeurs, les érythèmes solaires et les brûlures légères ainsi que pour les piqûres d'insectes. Elles sont utiles en hygiène corporelle locale par leurs propriétés calmantes, désinfectantes, cicatrisantes et anti-exudatives. En plus de la description qui précède un exemple de réalisation de la présente invention est rapporté ci-après. Cet exemple est donné à titre purement illustratif et non limitatif.

EXEMPLE.

| Acide borique | 0,8000 g |
|---|---|
| Acide salicylique | 0,2500 g |
| Tanin | 0,0250 g |
| Para-crésol | 0,0250 g |
| Glycérine | 0,0250 g |
| Teinture eucalyptus | 0,0250 g |
| Menthol | 0,0005 g |
| Eucalyptol | 0,0005 g |
| Eau pure | q.s.   100,0 g |

Indépendamment de leur activité, les lotions proposées sont avantageusement stables durant leur conservation et ne tachent pas les textiles.

Ainsi, la stabilité de la nouvelle lotion préférée de l'exemple ci-dessus a été étudiée de façon comparative à celle d'une lotion de soins de l'épiderme contenant entre autres constituants du résorcinol (métadiphénol). Dans cette étude, les solutions sont conservées à température ambiante et à l'abri de la lumière, l'évolution de leur coloration après leur fabrication au temps "$t_0$" est mesurée par spectrophotométrie à 430 nm en cuve de quartz de 1cm de trajet optique, l'eau étant utilisée comme liquide de compensation.

Les densités optiques présentées aux différentes périodes de l'étude sont reportées au tableau ci-dessous

| Temps | | Lotion de l'exemple | Lotion résorcinée |
|-------|---|---------------------|-------------------|
| $t_0$ | + 8 mois | D. opt.= 0,05 | D. opt.= 0,05 |
| $t_0$ | + 29 mois | | " = 0,45 |
| $t_0$ | + 36 mois | " = 0,20 | " = 0,70 |

Ainsi, on constate que la coloration de la nouvelle lotion suivant l'invention se modifie peu après 3 années de conservation par rapport à une solution d'hygiène contenant du résorcinol. Ceci reflète la stabilité de la solution qui est essentielle pour les traitements, généralement de longue durée, auxquels elle est destinée.

**Revendications**

1. Application d'une lotion aqueuse d'acide borique, d'acide salicylique et contenant, à titre de composé phénolique, uniquement un monophénol pour prévenir la chute des cheveux ou pour les faire repousser, à l'exception d'une application à titre de méthode de traitement du corps humain ou animal et de méthode de diagnostic appliquée au corps humain.

2. Application suivant la revendication 1, caractérisée en ce que la lotion contient de 0,3 à 3,0 % d'acide borique, de 0,1 à 1,0 % d'acide salicylique et de 0,001 à 0,1 % de monophénol par rapport à son poids total.

3. Application suivant la revendication 1 ou 2, caractérisée par une proportion pondérale relative de deux à vingt parties d'acide borique pour une partie d'acide salicylique.

4. Application suivant l'une des revendications 1 à 3, caractérisée en ce que la lotion contient de 0,01 à 0,10 % en poids d'acide tannique.

5. Application suivant l'une des revendications 1 à 4, caractérisée en ce que la lotion contient un excipient glycéro éthanolique à base de menthol et d'eucalyptol.

6. Lotion constituée d'une solution aqueuse d'acide borique, d'acide salicylique, d'un composé phénolique autre qu'un composé salicylique et, de préférence, d'un agent astringent, notamment d'acide tannique, caractérisée en ce que le composé phénolique autre qu'un composé salicylique est uniquement le p. crésol.

7. Lotion suivant la revendication 6 ou 7, caractérisée en ce que le p-crésol représente de 0,005 à 0,1 % du poids total de la lotion.

8. Lotion suivant la revendication 6 ou 7, caractérisée en ce qu'elle est constituée en poids par 0,50 à 1,20 % d'acide borique, 0,10 à 0,50 % d'acide salicylique, 0,010 à 0,050 % d'acide tannique, 0,010 à 0,050 % de para crésol et 0,01 à 0,10 % d'une solution glycéro-éthanolique de menthol et d'eucalyptol.

9. Procédé de préparation d'une lotion suivant la revendication 6, 7 ou 8, caractérisée en ce qu'il consiste à en mélanger les constituants.

EP 0 463 937 B1

**Claims**

1. Use of an aqueous boric acid and salicylic acid lotion containing, as a phenolic compound, only one monophenol, to prevent hair loss or to make it grow again, with the exception of use as a method of treating the human or animal body or as a method of diagnosis applied to the human body.

2. Use according to claim 1, characterised in that the lotion contains 0.3 to 3.0% of boric acid, 0.1 to 1.0% of salicylic acid and 0.001 to 0.1% of monophenol relative to its total weight.

3. Use according to claim 1 or 2, characterised by a relative proportion by weight of two to twenty parts of boric acid to one part of salicylic acid.

4. Use according to one of claims 1 to 3, characterised in that the lotion contains 0.01 to 0.10% by weight of tannic acid.

5. Use according to one of claims 1 to 4, characterised in that the lotion contains a glycero-ethanolic excipient based on menthol and eucalyptol.

6. Lotion consisting of an aqueous solution of boric acid, salicylic acid, a phenolic compound other than a salicylic compound and, preferably, an astringent, more particularly tannic acid, characterised in that the phenolic compound other than a salicylic compound is exclusively p-cresol.

7. Lotion according to claim 6 or 7, characterised in that the p-cresol represents from 0.005 to 0.1% of the total weight of the lotion.

8. Lotion according to claim 6 or 7, characterised in that it consists, by weight, of 0.50 to 1.20% of boric acid, 0.10 to 0.50% of salicylic acid, 0.010 to 0.050% of tannic acid, 0.010 to 0.050% of para-cresol and 0.01 to 0.10% of a glycero-ethanolic solution of menthol and eucalyptol.

9. Process for preparing a lotion according to claim 6, 7 or 8, characterised in that it comprises mixing the constituents.

**Patentansprüche**

1. Verwendung einer wässrigen Lösung mit Borsäure, Salicylsäure und einer Phenolverbindung, wobei als Phenolverbindung ausschließlich eine Monophenolverbindung eingesetzt wird, zur Vorbeugung gegen Haarausfall und zur Förderung des Nachwachsens der Haare, jedoch ausgenommen eine Verwendung zu Zwecken der Behandlung des menschlichen oder tierischen Körpers oder zu Zwecken einer Diagnose am menschlichen Körper.

2. Verwendung nach Anspruch 1,
dadurch gekennzeichnet, daß
die Lösung - bezogen auf ihr Gesamtgewicht - 0,3 bis 3,0 % Borsäure, 0,1 bis 1,0 % Salicylsäure und 0,001 bis 0,1 % Monophenolverbindungen enthält.

3. Verwendung nach Anspruch 1 oder 2,
dadurch gekennzeichnet, daß
2 bis 20 Gewichts-Teile Borsäure auf einen Gewichts-Teil Salicylsäure vorhanden sind.

4. Verwendung nach einem der Ansprüche 1 bis 3,
dadurch gekennzeichnet, daß
die Lösung zusätzlich 0,01 bis 0,10 Gew.-% Tanninsäure enthält.

5. Verwendung nach einem der Ansprüche 1 bis 4,
dadurch gekennzeichnet, daß
die Lösung einen Zusatz von Menthol und Eukalyptol in Glyzerin/Aethanol-Lösung enthält.

5

6.  Lotion, insbesondere Haarwasser
    bestehend aus einer wässrigen Lösung mit Borsäure, Salicylsäure und einer anderen Phenolverbindung als Salicylverbindung und vorzugsweise mit einem Adstringens, insbesondere Tanninsäure,
    dadurch gekennzeichnet, daß
    die Phenolverbindung, die keine Salicylverbindung ist, ausschließlich p-Kresol ist.

7.  Lotion, insbesondere Haarwasser nach Anspruch 6,
    dadurch gekennzeichnet, daß
    der Anteil an p-Kresol 0,005 bis 0,1 % vom Gesamtgewicht der Lotion bzw. des Haarwassers ausmacht.

8.  Lotion, insbesondere Haarwasser nach Anspruch 6 oder 7,
    dadurch gekennzeichnet, daß
    die wässrige Lösung 0,50 bis 1,20 Gew.-% Borsäure, 0,10 bis 0,50 Gew.-% Salicylsäure, 0,010 bis 0,050 Gew.-% Tanninsäure, 0,010 bis 0,050 Gew.-% Parakresol und 0,01 bis 0,10 Gew.-% einer Lösung von Menthol und Eukalyptol in Glyzerin/Aethanol enthält.

9.  Verfahren zur Herstellung einer Lotion, insbesondere eines Haarwassers nach Anspruch 6, 7 oder 8,
    dadurch gekennzeichnet,
    daß die Bestandteile der Lotion, insbesondere des Haarwassers miteinander vermischt werden.